# EUROPEAN PATENT APPLICATION

(11) **EP 3 875 091 A1**
(43) Date of publication of application: **08.09.2021**
(21) Application number: 19880661.4
(22) Date of filing: 31.10.2019
(51) Int. Cl.: A61K 31/505, A61K 9/48, A61K 47/12, A61K 47/26, A61K 47/38, A61P 35/00, A61P 35/02

(54) **PHARMACEUTICAL COMPOSITION CONTAINING ANTITUMOR AGENT**

(30) Priority: 31.10.2018 JP 2018204851
(71) Applicant: FUJIFILM Corporation, Tokyo 106-8620 (JP)
(72) Inventor: OURA Tai, Toyama-shi, Toyama 930-8508 (JP)
(74) Representative: Hoffmann Eitle
(86) International application number: PCT/JP2019/042750
(87) International publication number: WO 2020/090970

(57) **Abstract**

An object of the present invention is to provide a pharmaceutical composition containing a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide which is rapidly dissolved in a weakly acidic test solution. According to the present invention, a pharmaceutical composition containing a succinate salt of (S,E)-N-(1-((5-(2-((4-cyanophenyl) amino)-4-(propylamino)pyrimidin-5-yl)-4-pentyn-1-yl)ami no)-1-oxopropan-2-yl)-4-(dimethylamino)-N-methyl-2-butenamide and at least one or more kinds selected from the group consisting of celluloses, sugars, and sugar alcohol is provided.

## Description

### Field of the Invention

The present invention relates to a pharmaceutical composition excellent in dissolution property, which contains a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide (hereinafter, referred to as Compound A).

### Description of the Related Art

Compound A is a medically useful compound that has an FLT3 inhibitory action and has a strong effect on hematological cancer such as acute myeloid leukemia (Patent Document 1). In the medical field, there is a demand on a highly effective and stable pharmaceutical drug, which is expected to be applied clinically in a wide range of administration formulation forms such as an oral agent, an injection agent, an ointment, and a suppository.

On the other hand, with the advancement of in-home medical care and the promotion of self-medication, the maintenance of medication adherence is an important issue in drug therapy. The most commonly used formulation form in-home medical care is an oral agent (Non-Patent Document 1).

In a case where a formulation is orally administered, the formulation first enters the stomach and then in the process of migrating from the stomach to the small intestine, disintegrates and disperses, and the dissolution of the drug contained in the formulation occurs. The average pH in the stomach temporarily rises to around pH 4 during the day, which coincides with the time of having a meal; however, the pH drops after about 30 minutes from the time of having a meal and a low pH of around pH 2 is exhibited between meals (Non-Patent Document 2). On the other hand, it has been considered that in general, the acidity of gastric juice in healthy adults is high and is around pH 1; however, it has been clear that the proportion of adults whose gastric acid secretion is reduced increases as the age increases (Non-Patent Document 3). That is, the dissolution property in a weakly acidic test solution is one of the important indicators of a formulation. Generally, a formulation in which 85% or more of a drug is dissolved within 30 minutes is considered to have a rapid dissolution property.

### Prior Art Documents

### Patent Documents

Patent Document 1: WO2015/056683A

### Non-Patent Documents

Non-Patent Document 1: Japanese Journal of Pharmaceutical Health Care and Sciences, Vol. 34, No. 3, pp. 289 to 296, 2008
Non-Patent Document 2: Journal of Japanese Society of Gastroenterology, Vol. 85, No. 7, pp. 1353 to 1359, 1988
Non-Patent Document 3: Bioequivalence test of pharmaceutical products - Guideline compliance -, edited by Hiroyasu Ogata, p. 35, 2013

### SUMMARY OF THE INVENTION

The pharmaceutical composition produced by a conventional method containing the succinate salt of Compound A showed a low dissolution property in a dissolution test with a test solution having a pH of 4.5, where the test solution was a weakly acidic test solution.

In order to prevent a decrease in bioavailability, a pharmaceutical composition containing a succinate salt of Compound A that is rapidly dissolved in a weakly acidic test solution is strongly desired.

Under such circumstances, the inventors of the present invention carried out extensive studies and have found that a pharmaceutical composition containing a specific excipient (celluloses, sugars, or sugar alcohol) has an effect of improving dissolution in a weakly acidic solution. The present invention provides a pharmaceutical composition having a rapid dissolution property, which contains a succinate salt of Compound A.

The present invention provides the followings.
<1> A pharmaceutical composition comprising a succinate salt of (S,E)-N-(1-((5-(2-((4-cyanophenyl) amino)-4-(propylamino)pyrimidin-5-yl)-4-pentyn-1-yl)ami no)-1-oxopropan-2-yl)-4-(dimethylamino)-N-methyl-2-butenamide and at least one or more kinds selected from the group consisting of celluloses, sugars, and sugar alcohol.
<2> The pharmaceutical composition according to <1>, in which the celluloses are microcrystalline cellulose, the sugars are lactose, and the sugar alcohol is erythritol or mannitol.
<3> The pharmaceutical composition according to <1> or <2>, further comprising a lubricant.
<4> The pharmaceutical composition according to <3>, in which the lubricant is magnesium stearate or sodium stearyl fumarate.
<5> The pharmaceutical composition according to any one of <1> to <4>, in which the pharmaceutical composition is produced by a dry-type granulation method.
<6> The pharmaceutical composition according to any one of <1> to <5>, in which the pharmaceutical composition is a hard capsule.

The pharmaceutical composition containing a succinate salt of Compound A according to an aspect of the present invention is an excellent pharmaceutical composition having a rapid dissolution property even in a weakly acidic test solution.

### DESCRIPTION OF THE PREFERRED EMBODIMENTS

The present invention will be described in detail below.

As used in the present specification, % means the mass percentage unless otherwise particularly specified. In the present invention, the numerical range indicated by using "to" indicates a range including numerical values described before and after "to" as a minimum value and a maximum value, respectively. In addition, in the present invention, in a case where there are a plurality of substances corresponding to each component in the composition, the amount of each component in the composition means the total amount of the plurality of substances present in the composition unless otherwise particularly specified.

The pharmaceutical composition according to the embodiment of the present invention is a pharmaceutical composition containing a succinate salt of Compound A and at least one or more kinds selected from the group consisting of celluloses, sugars, and sugar alcohol.

The pharmaceutical composition according to the embodiment of the present invention has an excellent dissolution property; for example, in the dissolution test of the 17th revised Japanese Pharmacopoeia dissolution test method (the paddle method), the dissolution rate after 30 minutes is 85% by mass or more, where the dissolution test is carried out using an acetate buffer solution having a pH of 4.5 as the test solution under the condition of the rotation speed of 50 rotations/minute.

A succinate salt of Compound A used in the present invention can be produced, for example, by the method disclosed in WO2015/056683A.

Examples of celluloses which is used in the present invention include a water-insoluble cellulose derivative and a water-soluble cellulose derivative, and preferred examples thereof include a water-insoluble cellulose derivative.

Examples of the water-soluble cellulose derivative include hypromellose, hydroxypropyl cellulose, and sodium carboxymethyl cellulose.

Examples of the water-insoluble cellulose derivative include a microcrystalline cellulose, a powdered cellulose, ethyl cellulose, carmellose calcium, croscarmellose sodium, and low-substituted hydroxypropylcellulose, preferred examples thereof include a microcrystalline cellulose and a powdered cellulose, and more preferred examples thereof include a microcrystalline cellulose.

Examples of the microcrystalline cellulose include CEOLUS KG-1000 (Asahi Kasei Corporation), CEOLUS PH101 (Asahi Kasei Corporation), and CEOLUS PH-F20JP (Asahi Kasei Corporation), and preferred examples thereof include CEOLUS KG-1000 (Asahi Kasei Corporation).

Examples of the sugars which is used in the present invention include sucrose, lactose, maltose, and glucose, preferred examples thereof include lactose and maltose, and more preferred examples thereof include lactose.

Further, examples of the lactose include hydrated lactose, unhydrated lactose, a spray-dried hydrated lactose, and a granulated hydrated lactose.

Examples of the sugar alcohol which is used in the present invention include mannitol, erythritol, and xylitol, preferred examples thereof include mannitol and erythritol, and more preferred examples thereof include erythritol.

These celluloses, sugars, and sugar alcohol may be used alone or in a combination of two or more thereof.

The combined content of celluloses, sugars, and sugar alcohol may be 5% to 95%, preferably 10% to 90%, and more preferably 15% to 75%, with respect to the composition.

Examples of the lubricant which is used in the present invention include magnesium stearate, sodium stearyl fumarate, and hydrogenated oil, and preferred examples thereof include magnesium stearate and sodium stearyl fumarate.

Examples of magnesium stearate include Parteck™ LUB MST (Merck KGaA), magnesium stearate (vegetable) (Taihei Chemical Industrial Co., Ltd.), and Japanese Pharmacopeia Magnesium stearate JPM (Sakai Chemical Industry Co., Ltd.).

Examples of sodium stearyl fumarate include PRUV (JRS PHARMA).

The content of the lubricant may be 0.05% to 35%, preferably 0.5% to 20%, and more preferably 1% to 15%, with respect to the composition.

In the pharmaceutical composition according to the embodiment of the present invention, a pharmaceutically acceptable pharmaceutical aid can be used within the range in which the effects of the present invention are not impaired.

Examples of the pharmaceutical aid include a disintegrant, a binder, a sweetening agent, a coloring agent, a flavoring agent, a surfactant, a coating agent, and a plasticizer.

Examples of the disintegrant include carmellose, carmellose calcium, croscarmellose sodium, carboxymethyl starch sodium, crospovidone, low-substituted hydroxypropylcellulose, and partially pregelatinized starch.

Examples of the binder include hydroxypropyl cellulose, carmellose sodium, and methyl cellulose, hypromellose, and polyvinyl alcohol.

Examples of the sweetening agent include aspartame, saccharin, stevia, thaumatin, and acesulfame potassium.

Examples of the colorant include titanium dioxide, red ferric oxide, yellow ferric oxide, black iron oxide, Food Red No. 102, Food Yellow No. 4, and Food Yellow No. 5.

Examples of the flavoring agent include essential oils such as orange oil, lemon oil, peppermint oil, and pine oil; extracts such as orange extract and peppermint extract; flavors such as cherry flavor, vanilla flavor, and fruit flavor; powdered fragrances such as apple micron, banana micron, peach micron, strawberry micron, and orange micron; vanillin; and ethyl vanillin.

Examples of the surfactant include sodium lauryl sulfate, sodium dioctyl sulfosuccinate, polysorbate, and polyoxyethylene-hardened castor oil.

Examples of the coating agent include hypromellose, an aminoalkyl methacrylate copolymer E, an aminoalkyl methacrylate copolymer RS, ethyl cellulose, cellulose acetate phthalate, hydroxypropyl methyl cellulose phthalate, a methacrylic acid copolymer L, a methacrylic acid copolymer LD, and a methacrylic acid copolymer S.

Examples of the plasticizer include triethyl citrate, macrogol, triacetin, and propylene glycol.

These additives may be used alone or in a combination of two or more thereof.

The blending amount is not particularly limited, and blending may be carried out appropriately so that the effect thereof is sufficiently exhibited according to each purpose.

The pharmaceutical composition according to the embodiment of the present invention can be used as a pharmaceutical formulation such as a tablet, a hard capsule, granules, fine granules, powders, a fast-disintegrating tablet, a formulation dissoluble at use, a dry syrup, or a powder formulation, by appropriately using an excipient, a lubricant, and a pharmaceutical aid, which are pharmaceutically acceptable. A hard capsule or a tablet is referable, and a hard capsule is more preferable. Examples of the hard capsule include a hard capsule manufactured using gelatin, hypromellose, pullulan, or the like as a raw material, and preferred examples thereof include a hard capsule manufactured using hypromellose. Examples thereof include Vcaps Plus (Lonza Group AG) and Quali-V (Qualicaps Co., Ltd.). The size of the hard capsule is preferably No. 0 to No. 4 and more preferably No. 2 to No. 4.

The production of the pharmaceutical composition according to the embodiment of the present invention is not particularly limited and may be carried out by a conventional method.

Examples of the method for producing the pharmaceutical composition according to the embodiment of the present invention include a method of filling a hard capsule with a mixture of raw materials or tableting a mixture of raw materials. Another example thereof includes a method of granulating a mixture of raw materials and filling a hard capsule with the obtained granulated product or tableting the obtained granulated product.

Examples of the method for producing the pharmaceutical composition according to the embodiment of the present invention include the following method.
(1) A succinate salt of Compound A, a lubricant, and one or more additives among an excipient and a pharmaceutical aid are added and mixed to produce a mixed powder, the obtained mixed powder is compressed by a dry-type granulation method to produce a compression-molded product, and the obtained compression-molded product is crushed and sized to obtain a granulated product.
(2) An excipient and/or a pharmaceutical aid is added to the obtained granulated product and mixed to obtain a mixed powder.
(3-1) The obtained mixed powder is tableted to obtain an uncoated tablet. Further, the obtained uncoated tablet may be film-coated.
(3-2) A hard capsule is filled with the obtained granulated product to obtain a hard capsule.

Examples of the granulation method include a wet-type granulation method, a dry-type granulation method, and a melting granulation method, preferred examples thereof include a wet-type granulation method and a dry-type granulation method, and more preferred examples thereof include a dry-type granulation method.

Examples of the wet-type granulation method include a fluidized bed type granulation method, a wet-type crushing granulation method, a rotary granulation method, a spray-drying type granulation method, and an extrusion granulation method, preferred examples thereof include a fluidized bed type granulation method and the wet-type crushing granulation method, and more preferred examples thereof include a fluidized bed type granulation method.

Examples of the dry-type granulation method include a compacting method, a slugging method, and a briquetting method, and preferred examples thereof include a compacting method. Examples of the compacting method include a method of using a roller compactor. In a case where a roller compactor is used, the manufacturing pressure varies depending on the device type used; however, in a case where TF-LABO (manufactured by Freund Corporation) is used, 3 to 12 MPa is preferable.

### Examples

The usefulness of the pharmaceutical composition according to the embodiment of the present invention will be described in Examples and Comparative Examples; however, the present invention is not limited thereto.

In Examples and Comparative Examples, as a desktop V-type mixer, a VM-2 model manufactured by TSUTSUI Scientific Instruments Co., Ltd.;
as a dry-type granulator, TF-LABO manufactured by Freund Corporation was used;
as sodium stearyl fumarate, PRUV manufactured by JRS Pharma was used;
as magnesium stearate, Parteck™ LUB MST manufactured by Merck KGaA was used; and
as a hard capsule, Vcaps Plus made by Lonza Group AG was used.

### Example 1

20 g of a succinate salt of Compound A, 21.9 g of erythritol (Erythritol T fine powder, manufactured by Mitsubishi-Chemical Foods Corporation), and 0.6 g of sodium stearyl fumarate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Sodium stearyl fumarate was added to the obtained granulated product so that the content thereof was 0.2% of the total amount, and the resultant mixture was mixed with the desktop V-type mixer for 30 minutes. A No. 3 hard capsule was filled with 194.7 mg of the obtained mixed powder to obtain a hard capsule.

### Example 2

10 g of a succinate salt of Compound A, 10.9 g of lactose (Pharmatose 200M, manufactured by DFE Phrama), and 0.3 g of sodium stearyl fumarate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Sodium stearyl fumarate was added to the obtained granulated product so that the content thereof was 0.2% of the total amount, and the resultant mixture was mixed with the desktop V-type mixer for 30 minutes. A No. 3 hard capsule was filled with 194.7 mg of the obtained mixed powder to obtain a hard capsule.

### Example 3

10 g of a succinate salt of Compound A, 10.9 g of mannitol (Parteck M100, manufactured by Merck KGaA), and 0.3 g of sodium stearyl fumarate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Sodium stearyl fumarate was added to the obtained granulated product so that the content thereof was 0.2% of the total amount, and the resultant mixture was mixed with the desktop V-type mixer for 30 minutes. A No. 3 hard capsule was filled with 194.7 mg of the obtained mixed powder to obtain a hard capsule.

### Example 4

20 g of a succinate salt of Compound A, 21.9 g of microcrystalline cellulose (CEOLUS PH-F20JP, manufactured by Asahi Kasei Corporation), and 1.3 g of magnesium stearate were sieved through an 18 mesh sieve and then mixed with the desktop V-type mixer for 30 minutes. This mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.2% of the total amount, and the resultant mixture was mixed with the desktop V-type mixer for 30 minutes. A No. 3 hard capsule was filled with 197.8 mg of this mixed powder to obtain a hard capsule.

### Example 5

15 g of a succinate salt of Compound A, 8.2 g of microcrystalline cellulose (CEOLUS KG-1000, manufactured by Asahi Kasei Corporation), and 0.5 g of magnesium stearate were sieved through an 18 mesh sieve and then mixed with the desktop V-type mixer for 30 minutes. This mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.2% of the total amount, and the resultant mixture was mixed with the desktop V-type mixer for 30 minutes. A No. 3 hard capsule was filled with 144.6 mg of the obtained mixed powder to obtain a hard capsule.

### Example 6

20 g of a succinate salt of Compound A, 21.9 g of erythritol (Erythritol T fine powder, manufactured by Mitsubishi-Chemical Foods Corporation), and 0.4 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.2% of the total amount, and the resultant mixture was mixed with the desktop V-type mixer for 30 minutes. A No. 3 hard capsule was filled with 193.8 mg of this mixed powder to obtain a hard capsule.

### Comparative Example 1

20 g of a succinate salt of Compound A, 21.9 g of a powder maltose syrup (AMALTY MR, manufactured by Mitsubishi Corporation Life Sciences Limited), and 1.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.2% of the total amount, and the resultant mixture was mixed with the desktop V-type mixer for 30 minutes. A No. 3 hard capsule was filled with 197.8 mg of the obtained mixed powder to obtain a hard capsule.

### Comparative Example 2

20 g of a succinate salt of Compound A, 21.9 g of a powder maltose syrup (AMALTY MR, manufactured by Mitsubishi Corporation Life Sciences Limited), and 1.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Talc (CROWN TALC PP, manufactured by Matsumura Sangyo Co., Ltd.) was added to the obtained granulated product so that the content thereof was 1% of the total amount, and the resultant mixture was mixed with the desktop V-type mixer for 30 minutes. A No. 3 hard capsule was filled with 199.4 mg of this mixed powder to obtain a hard capsule.

### Comparative Example 3

20 g of a succinate salt of Compound A, 21.9 g of isomalt (galenIQ 801, manufactured by PALATINIT GmbH), and 1.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Magnesium stearate was added to the obtained granulated product so that the content thereof was 0.2% of the total amount, and the resultant mixture was mixed with the desktop V-type mixer for 30 minutes. A No. 3 hard capsule was filled with 197.8 mg of the obtained mixed powder to obtain a hard capsule.

### Comparative Example 4

20 g of a succinate salt of Compound A, 21.9 g of isomalt (galenIQ 801, manufactured by PALATINIT GmbH), and 1.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Talc (CROWN TALC PP, manufactured by Matsumura Sangyo Co., Ltd.) was added to the obtained granulated product so that the content thereof was 1% of the total amount, and the resultant mixture was mixed with the desktop V-type mixer for 30 minutes. A No. 3 hard capsule was filled with 199.4 mg of this mixed powder to obtain a hard capsule.

### Comparative Example 5

20 g of a succinate salt of Compound A, 21.9 g of hydrated trehalose (manufactured by Hayashibara Co., Ltd.), and 1.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Talc (CROWN TALC PP, manufactured by Matsumura Sangyo Co., Ltd.) was added to the obtained granulated product so that the content thereof was 1% of the total amount, and the resultant mixture was mixed with the desktop V-type mixer for 30 minutes. A No. 3 hard capsule was filled with 199.4 mg of the obtained mixed powder to obtain a hard capsule.

### Comparative Example 6

20 g of a succinate salt of Compound A, 21.9 g of sorbitol (Parteck SI 450, manufactured by Merck KGaA), and 1.3 g of magnesium stearate were sieved through an 18 mesh sieve and then mixed with the desktop V-type mixer for 30 minutes. The obtained mixed powder was compressed with the dry-type granulator to obtain a compression-molded product. The obtained compression-molded product was crushed and sized with an oscillator 16 mesh attached to the dry-type granulator to obtain a granulated product. Talc (CROWN TALC PP, manufactured by Matsumura Sangyo Co., Ltd.) was added to the obtained granulated product so that the content thereof was 1% of the total amount, and the resultant mixture was mixed with the desktop V-type mixer for 30 minutes. A No. 3 hard capsule was filled with 199.4 mg of the obtained mixed powder to obtain a hard capsule.

### Test example 1

As samples, the capsule agents of Examples 1 to 6 and Comparative Examples 1 to 6 were used.

The dissolution test was carried out by the Japanese Pharmacopoeia dissolution test method (the paddle method). The rotation speed of the paddle was 50 rotations/minute. The sample contained in the sinker was put into 900 mL of an acetate buffer solution having a pH of 4.5, the test solution was collected 30 minutes later, and the dissolution rate (%) of Compound A was determined by the absorbance method.

The results are shown in Table 1 and Table 2.

**[Table 1]**

| Unit (mg) | Example 1 | Example 2 | Example 3 | Example 4 | Example 5 | Example 6 |
|---|---|---|---|---|---|---|
| Succinate of Compound A | 91.5 | 91.5 | 91.5 | 91.5 | 91.5 | 91.5 |
| Erythritol | 100.0 | | | | | 100.0 |
| Lactose | | 100.0 | | | | |
| Mannitol | | | 100.0 | | | |
| Microcrystalline cellulose | | | | 100.0 | 50.0 | |
| Powder maltose syrup | | | | | | |
| Isomalt | | | | | | |
| Hydrated trehalose | | | | | | |
| Sorbitol | | | | | | |
| Magnesium stearate | | | | 6.3 | 3.1 | 2.3 |
| Sodium stearyl fumarate | 3.2 | 3.2 | 3.2 | | | |
| Talc | | | | | | |
| Total | 194.7 | 194.7 | 194.7 | 197.8 | 144.6 | 193.8 |
| Dissolution rate (%) | 100 | 102 | 99 | 85 | 91 | 94 |

**[Table 2]**

| Unit (mg) | Comparative Example 1 | Comparative Example 2 | Comparative Example 3 | Comparative Example 4 | Comparative Example 5 | Comparative Example 6 |
|---|---|---|---|---|---|---|
| Succinate of Compound A | 91.5 | 91.5 | 91.5 | 91.5 | 91.5 | 91.5 |
| Erythritol | | | | | | |
| Lactose | | | | | | |
| Mannitol | | | | | | |
| Microcrystalline cellulose | | | | | | |
| Powder maltose syrup | 100.0 | 100.0 | | | | |
| Isomalt | | | 100.0 | 100.0 | | |
| Hydrated trehalose | | | | | 100.0 | |
| Sorbitol | | | | | | 100.0 |
| Magnesium stearate | 6.3 | 5.9 | 6.3 | 5.9 | 5.9 | 5.9 |
| Sodium stearyl fumarate | | | | | | |
| Talc | | 2.0 | | 2.0 | 2.0 | 2.0 |
| Total | 197.8 | 199.4 | 197.8 | 199.4 | 199.4 | 199.4 |
| Dissolution rate (%) | 37 | 33 | 48 | 37 | 55 | 78 |

The pharmaceutical composition containing a succinate salt of Compound A and at least one or more kinds selected from the group consisting of celluloses, sugars, and sugar alcohol exhibited a rapid dissolution property.

The pharmaceutical composition according to the embodiment of the present invention is useful as a pharmaceutical composition having an excellent dissolution property, which contains a succinate salt of Compound A.

## Claims

1. A pharmaceutical composition comprising a succinate salt of (S,E)-N-(1-((5-(2-((4-cyanophenyl)amino)-4-(propylamino)pyrimidin-5-yl)-4-pentyn-1-yl)ami no)-1-oxopropan-2-yl)-4-(dimethylamino)-N-methyl-2-butenamide and at least one or more kinds selected from the group consisting of celluloses, sugars, and sugar alcohol.

2. The pharmaceutical composition according to claim 1, wherein the celluloses are microcrystalline cellulose,
the sugars are lactose, and
the sugar alcohol is erythritol or mannitol.

3. The pharmaceutical composition according to claim 1 or 2, further comprising a lubricant.

4. The pharmaceutical composition according to claim 3, wherein the lubricant is magnesium stearate or sodium stearyl fumarate.

5. The pharmaceutical composition according to any one of claims 1 to 4, wherein the pharmaceutical composition is produced by a dry-type granulation method.

6. The pharmaceutical composition according to any one of claims 1 to 5, wherein the pharmaceutical composition is a hard capsule.
